# EUROPEAN PATENT SPECIFICATION

(11) **EP 2 802 359 B1**
(45) Date of publication and mention of the grant of the patent: **24.02.2016**
(21) Application number: 12784676.4
(22) Date of filing: 29.09.2012
(51) Int. Cl.: A61L 2/18, A61L 2/24, A61B 1/12

(54) **MACHINE FOR COLD SANITATION TREATMENT OF MEDICAL DEVICES**
MASCHINE ZUR KALTDESINFEKTION VON MEDIZINISCHEN VORRICHTUNGEN
MACHINE DE TRAITEMENT DE DÉSINFECTION À FROID POUR DISPOSITIFS MÉDICAUX

(30) Priority: 29.09.2011 IT VI20110260
(43) Date of publication of application: 19.11.2014
(73) Proprietor: IMS S.r.l., 00040 Pomezia Rm (IT)
(72) Inventor: AFFAITATI, Pietro, I-00041 Albano Laziale (RM) (IT)
(86) International application number: PCT/IB2012/001918
(87) International publication number: WO 2013/046010

(56) References cited:
- WO-A1-97/32610
- WO-A1-2008/020770
- US-A- 5 882 589
- US-A- 5 906 802
- US-A1- 2009 104 094

## Description

### Technical Field

The present invention is generally applicable to the technical field of the cleaning, decontamination, sterilization and disinfection of reusable medical devices and has particularly for object a machine for the sanitizing treatment of rigid and flexible medical devices after their use on a patient.

### Background art

As is known, all medical devices designed to come into contact with the body of a patient during treatment and diagnosis require, before being re-used on a new patient, to be "reprocessed", i.e. to be subjected to a more or less thrust sanitizing treatment.

Such sanitizing treatment may be a simple disinfection treatment or a sterilization process, performed both hot and cold, also depending on the materials the various devices are made of.

For example, for endoscopes or other optical instruments generally sensitive to relatively high temperatures of disinfecting autoclaves, cold treatment is performed by using a washing liquid, generally water, comprising in solution one or more chemical agents with less or more high decontaminating/sterilizing properties. Typically, the machines for the sanitizing treatment of medical devices provide that the device to be treated is inserted into a basin designed to be filled with the washing liquid.

Some examples of this type of machine are disclosed in US7138087 and in WO2011069064, in which the sanitizing tanks are defined by one or more fixed compartments each adapted to house a flexible endoscope.

US5882589 discloses a further machine for sterilizing medical devices having a sanitizing basin adapted to house a single device. In particular, the basin has inside an elevated portion or island from which nozzles extend for supplying the sanitizing liquids and air and around which the device is arranged. A similar machine is also known from WO2008/020770.

From WO2005056060 and EP1901784, the latter in the name of the same Applicant, machines are known for the sanitizing treatment of endoscopes both type of rigid or flexible.

These machines require that the devices are placed in a container or movable case forming the sanitation basin.

However, in all these cases, the sanitizing basing is suitable to accommodate only one single type of flexible endoscope or one or more optical instruments of the rigid type.

In the latter case, moreover, the rigid optical instruments are not stably housed in the basin, with following risk of an incorrect treatment and possible damage in the event that the basin was defined by the removable container.

From WO97/32610 a further machine is known which is designed to treat simultaneously both flexible endoscopes and rigid endoscopes. In particular, this machine comprises a removable container for housing the rigid and flexible endoscopes designed to be immersed in a basin containing a disinfectant solution. This solution, however, turns out to be very complex and requires appropriate connections between the basin containing the solution and the container. In particular, the latter has a plurality of slots adapted to allow the passage of the solution thereinside. However, in this way the perfect sanitation of the internal channels of flexible endoscopes is not granted, as the disinfectant solution can not penetrate with effectiveness thereinside, due to their small diameter.

### Disclosure of the invention

The object of the present invention is to overcome the above mentioned drawbacks, by providing a machine for the sanitizing treatment of rigid and flexible medical devices that has properties of high efficiency and relative cost effectiveness.

A particular object is to provide a machine for the sanitizing treatment of medical devices that allows to treat simultaneously both flexible endoscopes and rigid optical instruments, always granting their stability in positioning and the respect of the operating parameters during the entire cycle of sanitation.

Such objects, and others which will become more apparent hereinafter, are obtained by a machine for the sanitizing treatment of rigid and flexible medical devices, according claim 1, comprising at least one sanitizing chamber having a first compartment for housing a first medical device, means for supplying inside said first compartment one or more sanitizing fluids contained in respective tanks, wherein the sanitizing chamber comprises a bottom wall for positioning the first medical device and a central portion which rises from said bottom wall to define with the inner side wall of said chamber said first compartment having a substantially annular shape. The machine is characterized by the fact that said risen central portion comprises a groove formed on its upper wall and adapted to define a second compartment for a second medical device, said groove being completely contained in said first compartment and being provided with at least one input and an outlet for allowing the circulation thereinside of the fluid supplied from said supplying means and allow the simultaneous sanitizing treatment of the first medical device housed in said first compartment and of the second medical device housed in said second compartment by means of the same sanitizing fluid.

Thanks to this particular combination of features, it is possible to house in the same sanitizing chamber both a flexible medical device, such as an endoscope, and a rigid medical device, such as an optical instrument for endoscopic diagnosis, to allow their simultaneous treatment, maintaining the rigid device always safely housed in the sanitizing chamber.

Advantageous embodiments of the invention are obtained according to the dependent claims.

### Brief disclosure of the drawings

Further characteristics and advantages of the invention will become more apparent in light of the detailed description of a preferred but not exclusive embodiment of a machine according to the invention, shown only by way of non-limiting example with the aid of the accompanying drawings in which:
FIG. 1 is an elevated view of a machine according to the invention;
FIG. 2 is an enlarged view of a first particular of the machine of Fig. 1;
la FIG. 3 is an enlarged view of a second particular of the machine of Fig. 1;
FIG. 4 represents the hydraulic scheme of the machine according to the invention in the embodiment of Fig. 1.

### Best mode of carrying out the invention

With reference to the above figures, there is shown a machine for the cold sanitizing treatment of reusable medical devices, both rigid and flexible, not shown in these figures as they are not part of the invention.

In particular, the machine may be used to treat both flexible endoscopes, of the type having a sheath surrounding one or more inner channels, and other optical instruments of the rigid type for diagnostic and/or surgical use.

The machine according to the invention, generally referred with 1, may be used to carry out a more or less advanced sanitizing treatment, or "reprocessing", of the devices, which may be treatments of simple cleansing or disinfection and/or sterilization, generally cold treatments, i.e. by means of working fluids already active at temperatures substantially close to the environment temperature.

More precisely, the level of disinfection or sterilization of the devices may be linked to the disinfectant/sterilizing properties of the chemical agents in solution or dispersed in a liquid carrier, generally water, and/or to the contact and immersion time of the devices within the said liquid.

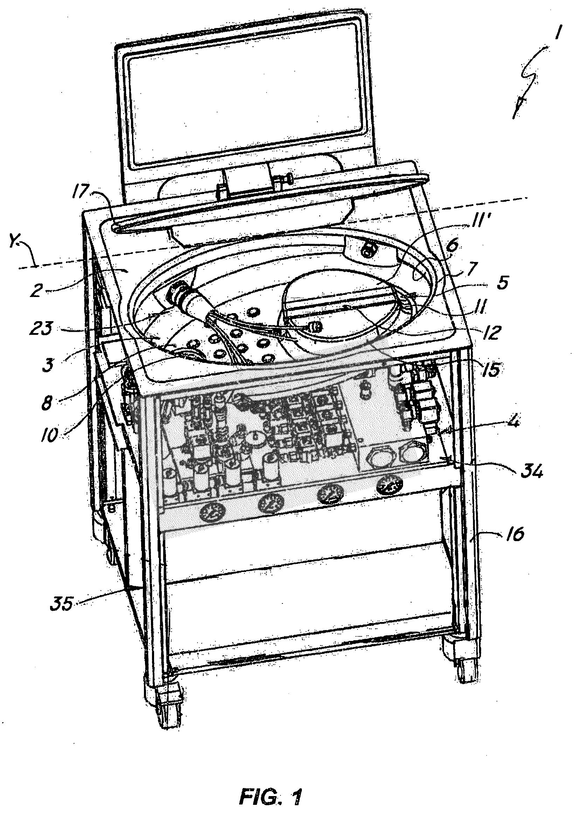

With reference to Figure 1, a machine 1 according to the invention will essentially comprise a sanitizing chamber 2 having a first compartment 3 for housing a flexible medical device to be treated and means 4 for supplying one or more sanitizing fluids inside of the first compartment 3.

According to a peculiar feature of the invention, the sanitizing chamber 2 also comprises a second compartment 5 fluidically connected with the first compartment 3 and adapted to stably house a rigid device, such as an endoscope or other rigid optical instrument for the sanitizing treatment thereof.

In particular, the machine 1 may be designed for the simultaneous sanitizing treatment of a flexible device and of a rigid device housed in the respective e compartments 3, 5.

However, the machine 1 can also be used to treat a medical device at a time, flexible or rigid, so as to allow the traceability of the entire operation. In any case, it will get the advantage of having a simple and compact machine suitable to treat both rigid and flexible medical devices using the same supplying means 4.

Suitably, the second compartment 5 will be completely contained in the first compartment 3 and the sanitizing chamber 2 may have a substantially annular peripheral portion defining the first compartment 3.

The peripheral portion has a side wall 6 with an upper opening 7 and a bottom wall 8 for positioning the flexible device.

In the preferred configuration illustrated, the supplying means 4 comprise a plurality of hydraulic circuits 9 each having an inlet 9' connectable to a respective tank S, S', S" and a plurality of outputs 9" made in the side wall 6 of the sanitizing chamber 2 for supplying the fluid in the first compartment 3.

According to alternative embodiments, not shown in the attached drawings, the outputs 9" for the fluids may also be formed at other zones of the first compartment 3 or of the chamber 2, for example at the bottom wall 8 or at an upper wall.

The bottom wall 8 of the sanitizing chamber 2 may also have a drain 10 for the exhausted fluids.

Advantageously, the sanitizing chamber 2 may include a substantially cylindrical or slightly tapered central portion 11, which rises from the bottom wall 8 and which has an upper front surface 11' having a radial groove 12 defining the second compartment 5.

Suitably, the groove 12 may extend radially and centrally on the upper front surface 11' and its dimensions in length, width and depth may be sized to allow the stable and removable housing of the common rigid optical instruments.

Suitably, the height of the risen central portion 11 will be less than the maximum height of the sanitizing chamber 2 so that the second compartment 5 is completely contained in the first compartment 3.

The groove 12 will be substantially elongated along a substantially radial direction X with opened ends 13, 14 for the fluidic connection with the first compartment 3.

In this way, the sanitizing flow will pass from the first compartment 3 to the second compartment 5 without the need to provide further connections.

The first compartment 3 will extend peripherally to the second compartment 5 so as to envelop it completely. The flexible device will be so positioned around the risen central portion 11, enabling the reduction of the overall dimensions of the machine 1. In a particular embodiment, also not illustrated, the outputs 9" for the fluids may be realized in correspondence of the side wall 15 of the risen central portion 11. Suitably, the sanitizing chamber 2 may be defined by a fixed basin formed within a supporting frame 16 which also house all hydraulic, electrical and electronic parts of the machine 1.

Additionally, the machine 1 may include an at least partially removable closure lid 17, for example hinged about a substantially horizontal axis Y, and adapted to seal the upper opening 7 of the sanitizing chamber 2, simultaneously closing both the first compartment 3 and the second compartment 5.

Conveniently, the machine 1 may be provided with control means, not shown in these figures, for selectively opening/closing the lid 17. For example, such control means may include a foot pedal adapted to control the opening and closing of the lid 17, so as to allow an operator to easily manipulate the devices. The control means may include means for the automatic opening/closing of the lid 17, possibly provided with a presence sensor, not shown, for enabling/disabling the opening/closing means, so as allow the movement of the lid 17 and the access to devices only in the presence of personnel.

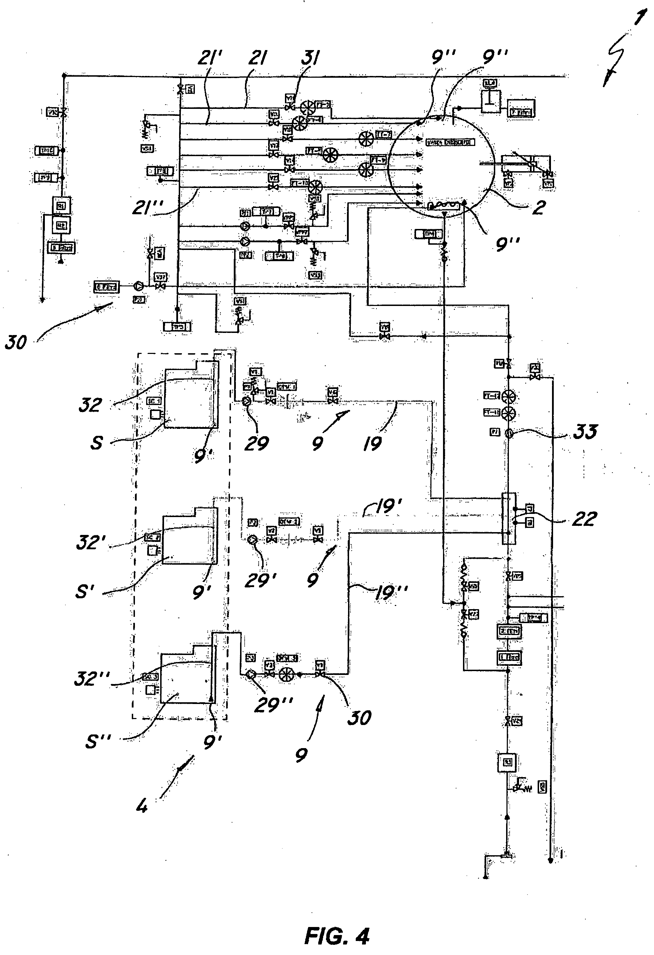

As schematically shown in Fig 4, each hydraulic circuit 9 will include a drawing conduit 19, 19', 19" each having an inlet 9' connectable to a respective tank S, S', S" for drawing up the sanitizing agents and one or more delivery conduits 20, 20', 20" connected to respective outputs 9" for supplying the fluids directly into the first compartment 3.

The supplying means 4 may also comprise a further supply conduit 21 connectable to the water mains to draw water to be mixed within a mixing chamber 22 with the chemical agents drawn from the tanks S, S', S" and obtain the sanitizing fluids.

The mixing chamber 22 may have at its input the drawing conduits 19, 19', 19" and the water supply conduit 21 while the output will have the delivery conduits 20, 20', 20", ...

Suitably, one or more outputs 9" may be designed to directly supply the fluid in the first compartment 3, while other outputs will be designed to be connected to the inner channels of the flexible device by appropriate means for fluidic connection 23. Generally, the fluidic connection means 23 comprise a plurality of connection pipes 24 having one end connected to a respective output 9 and the opposite end provided with a connector 25 coupled to an inner channel of the flexible device housed in the first compartment 3 for entering the sanitizing fluids thereinside.

In a first embodiment, not shown, the connecting pipes 24 will be of flexible type with small diameter and a first end connected directly to one of the outputs 9", in a fixed or removable manner, and the opposite end fitted with the connector 25, which will be of the traditional type and commonly available on the market, chosen according to the type of endoscope to be treated and the particular channel to be served.

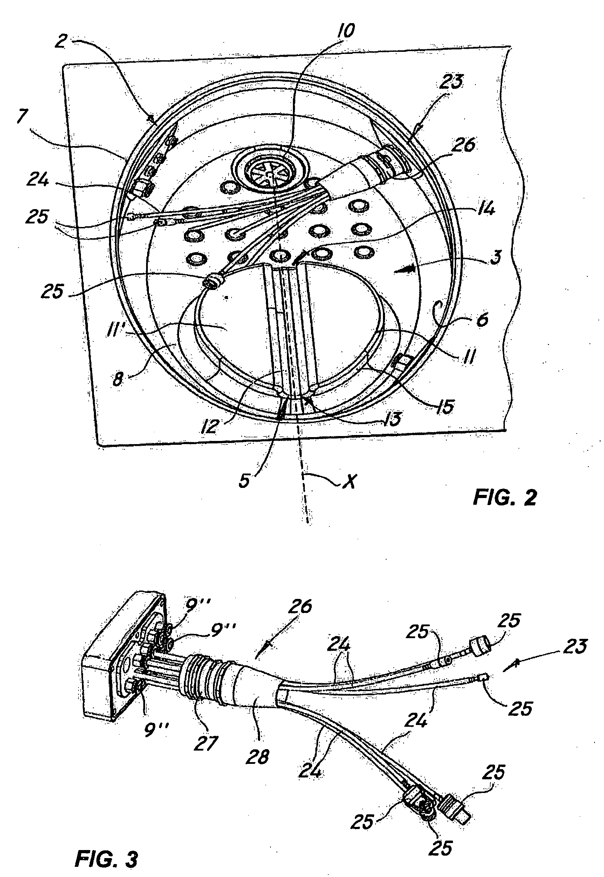

In the embodiment shown in greater detail in Fig 3, the fluidic connection means 23 comprise a quick coupling interface element 26 provided with a fixed portion 27 with a plurality of substantially tubular coupling elements, not visible in the figures, stably connected to the outputs 9" formed on the side wall 6 and a movable portion 28 integral with the connecting pipes 24.

The movable portion 28 will be designed to be stably and removably coupled to the fixed portion 27 so as to fluidically connect each of the pipes 24 to a respective output 9" through a respective coupling element.

Suitably, the two portions 27, 28 of the interface element 26 will be configured to connect the pipes 24 to the coupling elements in a univocal way, allowing the rapid, simultaneous and univocal fluidic connection of the internal channels of the device to the supplying means 4.

In known manner, the hydraulic circuits 9 may further comprise one or more first pumping devices 29, 29', 29" adapted to draw up the sanitizing agents from the respective tanks S, S', S" and to send them within the sanitizing chamber 2 through the drawing conduits 19, 19', 19" and delivery conduits 20, 20', 20", according to times and modes that can be adjusted by suitable programmable control means, not shown. The supplying means 4 may also include a circuit 30 for feeding compressed air, for example at a pressure ranging between 2 and 4 bar, connectable with the inside of the sheath of the endoscope for the execution of a leak test, according to known methods.

Suitably, the supplying means 4 may comprise means for adjusting the flow rate associated with each of the circuits 9 and for example defined by a plurality of solenoid valves 31 so as to be able to enable/disable the same in a selective and reciprocally independent manner, as a function of the sanitizing cycle to be performed.

Each drawing conduit 19, 19', 19" may include a picking spear 32, 32', 32" designed to be inserted into a respective tank S, S', S", immersed in the fluid chemical agent, and a first pumping device 29, 29', 29" adapted to promote the circulation of the fluid from the respective tank S, S', S" to the mixing chamber 22.

A second pumping device 33 may supply the fluid mixture from the mixing chamber 22 to the sanitizing chamber 2.

Suitably, the electrical components of the electronic and electromechanical supplying means 4 may be arranged in a first housing 34 placed immediately below the sanitizing chamber 2.

In addition, there will be a second housing 35 for the tanks S, S', S", placed downwardly to the first housing 34 and possibly joined to a third housing for the filter, not shown.

In the shown configuration there is a first tank S for a detergent and decontaminating solution containing a multi-enzymatic mixture synergized with a molecule of adazone ®, a second tank S' for a first sterilizing solution with a 5% solution of peracetic acid and a third tank S" containing a second sterilizing solution containing adazone ® and adapted to cooperate with the first to activate it. Still by way of example, the sterilizing molecule cited above may be that described in European patent EP1059292 or in European patent application EP2099501, both in the name of the same Applicant. One of the solutions may be the one described in the Italian patent application RM2005A000597 of the same Applicant.

From the above description it is evident that the invention achieves the intended object and in particular to make available a machine for the simultaneous sanitizing treatment of flexible and rigid medical devices which is particularly simple and compact and which always ensures the correct positioning of the devices to be treated.

The machine according to the invention is susceptible of numerous modifications and variations, all falling within the inventive concept expressed in the appended claims.

Even if the machine has been disclose with particular reference to the attached figures, reference numbers used in the description and in the claims are used to improve the intelligence of the invention and do not constitute any limitation to the scope of claimed protection.

## Claims

1. A machine for cold sanitation treatment of medical devices, comprising:
- at least one sanitation chamber (2) having a first compartment (3) for housing a first medical device;
- means (4) for supplying one or more sanitation fluids contained into respective tanks (S, S', S) inside said first compartment (3);
wherein said sanitation chamber (2) comprises a bottom wall (8) for positioning the first medical device and a central portion (11) which raises from said bottom wall (8) to define with the inner side wall (6) of said chamber (2) said first compartment (3) having a substantially annular shape;
**characterized in that** said risen central portion (11) comprises a slot (12) formed on its upper front surface (11') and adapted to define a second compartment (5) for a second medical device, said slot (12) being completely contained into said first compartment (3) and provided with at least one inlet (14) and one outlet (13) for allowing the circulation thereinto of the fluid supplied by said supplying means (4) and allowing the simultaneous sanitizing treatment of the first medical device housed in said first compartment (3) and of the second medical device housed into said second compartment (5) by means of the same sanitizing fluid.

2. Machine according claim 1, **characterized in that** said risen central portion (11) of said sanification chamber (3) is substantially cylindrical, said slot (12) extending on said upper surface (11') in a substantially radial direction with opened ends (13,14) for the fluidic connection with said first compartment (3).

3. Machine according claim 2, **characterized in that** said slot (12) is upperly opened, said side wall (6) of said sanitation chamber (2) having an upper opening (7) adapted to allow the access both to said first compartment (3) and to said second compartment (5).

4. Machine according claim 3, **characterized in that** said sanitation chamber (2) comprises an at least partially removable closing lid (17) adapted to close said upper opening (7) of said chamber (2) to simultaneously avoid the access to said first compartment (3) and to said second compartment (5).

5. Machine according claim 4, **characterized in that** it comprises a control pedal for the selective opening/closing of said lid (17).

6. Machine according claim 4, **characterized in that** it comprises means for the automatic opening/closing of said lid (17) provided with a proximity sensor for enabling/disabling said opening/closing means.

7. Machine according any preceding claim, **characterized in that** said supply means (4) comprise a plurality of hydraulic circuits (9) each having an inlet (9') connectable to a respective tank (S, S', S") and a plurality of outlets (9") realized into said side wall (6) of said sanitation chamber (2) for the fluids flow into said first compartment (3).

8. Machine according to claim 7, **characterized in that** it comprises a plurality of connection pipes (24) having one end connected to a respective of said outlets (9") and the opposite end provided with a connector (25) coupable to an inner channel of a flexible device housed into said first compartment (3) for the sanitation fluids to flow thereinside.

9. Machine according claim 4, **characterized in that** said bottom wall (8) of said sanitation chamber (2) has a drain (10) for the exhausted fluids.

## Patentansprüche

1. Maschine zur Kaltreinigungsbehandlung medizinischer Geräte, umfassend:
- mindestens eine Reinigungskammer (2) mit einem ersten Fach (3) zum Beherbergen eines ersten medizinischen Geräts,
- Mittel (4) zum Zuführen eines oder mehrerer Reinigungsfluide, die in entsprechenden Tanks (S, S', S) im Inneren des ersten Fachs (3) enthalten sind,
wobei die Reinigungskammer (2) eine Bodenwand (8) zum Anordnen des ersten medizinischen Geräts und einen zur Bodenwand (8) erhöhten mittleren Abschnitt (11) umfasst, um mit der Innenseitenwand (6) der Kammer (2) das erste Fach (3) mit einer im Wesentlichen ringförmigen Form zu bilden,
**dadurch gekennzeichnet, dass** der erhöhte mittlere Abschnitt (11) einen Schlitz (12) umfasst, der in seiner oberen Vorderseite (11') gebildet und dafür eingerichtet ist, ein zweites Fach (5) für ein zweites medizinisches Gerät zu bilden, wobei der Schlitz (12) vollkommen in dem ersten Fach (3) enthalten und mit mindestens einem Einlass (14) und einem Auslass (15) versehen ist, um die Zirkulation des von den Zuführungsmitteln (4) zugeführten Fluids zu ermöglichen und die gleichzeitige Reinigungsbehandlung des im ersten Fach (3) untergebrachten ersten medizinischen Geräts und des im zweiten Fach (5) untergebrachten zweiten medizinischen Geräts durch dasselbe Reinigungsfluid zu ermöglichen.

2. Maschine nach Anspruch 1, **dadurch gekennzeichnet, dass** der erhöhte mittlere Abschnitt (11) der Reinigungskammer (2) im Wesentlichen zylindrisch ist, wobei sich der Schlitz (12) auf der Oberseite (11') in einer im Wesentlichen radialen Richtung mit geöffneten Enden (13, 14) zur Fluidverbindung mit dem ersten Fach (3) erstreckt.

3. Maschine nach Anspruch 2, **dadurch gekennzeichnet, dass** der Schlitz (12) oben geöffnet ist und die Seitenwand (6) der Reinigungskammer (2) eine obere Öffnung (7) aufweist, die dafür eingerichtet ist, den Zugang sowohl zum ersten Fach (3) als auch zum zweiten Fach (5) zu ermöglichen.

4. Maschine nach Anspruch 3, **dadurch gekennzeichnet, dass** die Reinigungskammer (2) einen mindestens teilweise abnehmbaren Deckel (17) umfasst, der dafür eingerichtet ist, die obere Öffnung (7) der Kammer (2) zu verschließen, um gleichzeitig den Zugang zum ersten Fach (3) und zum zweiten Fach (5) zu verhindern.

5. Maschine nach Anspruch 4, **dadurch gekennzeichnet, dass** sie ein Steuerpedal zum selektiven Öffnen/Schließen des Deckels (17) umfasst.

6. Maschine nach Anspruch 4, **dadurch gekennzeichnet, dass** sie Mittel zum automatischen Öffnen/Schließen des Deckels (17) umfasst, die mit einem Näherungssensor zum Aktivieren/Deaktivieren der Mittel zum Öffnen/Schließen versehen sind.

7. Maschine nach einem vorhergehenden Anspruch, **dadurch gekennzeichnet, dass** die Zuführungsmittel (4) mehrere Hydraulikkreise (9) umfasst, die jeweils einen Einlass (9'), der an einen entsprechenden Tank (S, S', S") anschließbar ist, und mehrere Auslässe (9") aufweisen, die in der Seitenwand (6) der Reinigungskammer (2) realisiert sind, damit die Fluide in das erste Fach (3) strömen.

8. Maschine nach Anspruch 7, **dadurch gekennzeichnet, dass** sie mehrere Anschlussrohre (24) umfassen, deren eines Ende an einen entsprechenden Auslass (9") angeschlossen ist und deren gegenüberliegendes Ende mit einem Anschlussstück (25) versehen ist, das mit einem inneren Kanal einer flexiblen Vorrichtung koppelbar ist, die in dem ersten Fach (3) untergebracht ist, damit die Reinigungsfluide in dieses einströmen können.

9. Maschine nach Anspruch 4, **dadurch gekennzeichnet, dass** die Bodenwand (8) der Reinigungskammer (2) einen Abfluss (10) für die verbrauchten Fluide aufweist.

## Revendications

1. Machine pour le traitement de désinfection à froid de dispositifs médicaux, comprenant :
- au moins une chambre de désinfection (2) ayant un premier compartiment (3) pour loger un premier dispositif médical ;
- un moyen (4) pour alimenter un ou plusieurs fluides de désinfection contenus dans des réservoirs respectifs (S, S', S") à l'intérieur dudit premier compartiment (3) ;
dans laquelle ladite chambre de désinfection (2) comprend une paroi de fond (8) pour positionner le premier dispositif médical et une portion centrale (11) qui s'élève de ladite paroi de fond (8) pour définir avec la paroi latérale interne (6) de ladite chambre (2) ledit premier compartiment (3), qui a une forme sensiblement annulaire ;
**caractérisée en ce que** ladite portion centrale (11) surélevée comprend une rainure (12) formée sur sa surface frontale supérieure (11') et adaptée pour définir un deuxième compartiment (5) pour un deuxième dispositif médical, ladite rainure (12) étant complètement contenue dans ledit premier compartiment (3) et pourvue d'au moins une entrée (14) et une sortie (15) pour y permettre la circulation du fluide alimenté par ledit moyen d'alimentation (4) et permettant le traitement de désinfection simultané du premier dispositif médical logé dans ledit premier compartiment (3) et du deuxième dispositif médical logé dans ledit deuxième compartiment (5) à l'aide du même fluide de désinfection..

2. Machine selon la revendication 1, **caractérisée en ce que** ladite portion centrale surélevée (11) de ladite chambre de désinfection (3) est sensiblement cylindrique, ladite rainure (12) s'étendant sur ladite surface supérieure (11') en direction sensiblement radiale avec des extrémités ouvertes (13, 14) pour la connexion fluide avec ledit premier compartiment (3).

3. Machine selon la revendication 2, **caractérisée en ce que** ladite rainure (12) est ouverte dans sa partie supérieure, ladite paroi latérale (6) de ladite chambre de désinfection (2) ayant une ouverture supérieure (7) adaptée pour permettre l'accès aussi bien audit premier compartiment (3) qu'audit deuxième compartiment (5).

4. Machine selon la revendication 3, **caractérisée en ce que** ladite chambre de désinfection (2) comprend un couvercle de fermeture au moins partiellement amovible (17) adapté pour fermer ladite ouverture supérieure (7) de ladite chambre (2) pour éviter simultanément l'accès audit premier compartiment (3) et audit deuxième compartiment (5).

5. Machine selon la revendication 4, **caractérisée en ce qu'**elle comprend une pédale de commande pour l'ouverture/fermeture sélective dudit couvercle (17).

6. Machine selon la revendication 4, **caractérisée en ce qu'**elle comprend un moyen pour ouvrir/fermer automatiquement ledit couvercle (17) pourvu d'un capteur de proximité pour habiliter/inhabiliter ledit moyen d'ouverture/fermeture.

7. Machine selon l'une quelconque des revendications précédentes, **caractérisée en ce que** ledit moyen d'alimentation (4) comprend une pluralité de circuits hydrauliques (9) ayant chacun une entrée (9') connectable à un réservoir respectif (S, S', S") et une pluralité de sorties (9") réalisées dans ladite paroi latérale (6) de ladite chambre de désinfection (2) pour l'écoulement des fluides dans ledit premier compartiment (3).

8. Machine selon la revendication 7, **caractérisée en ce qu'**elle comprend une pluralité de tubes de connexion (24) ayant une extrémité connectée à l'une desdites sorties respectives (9") et l'extrémité opposée pourvue d'un connecteur (25) connectable à un canal interne d'un dispositif flexible logé dans ledit premier compartiment (3) pour l'écoulement de fluides de désinfection.

9. Machine selon la revendication 4, **caractérisée en ce que** ladite paroi de fond (8) de ladite chambre de désinfection (2) comporte un drain (10) pour les fluides usés.
